# EUROPEAN PATENT APPLICATION

(11) **EP 4 803 602 A1**
(43) Date of publication of application: **09.09.2026**
(21) Application number: 24885588.4
(22) Date of filing: 24.10.2024
(51) Int. Cl.: C12M 1/00, G01N 37/00

(54) **FLUID DEVICE**

(30) Priority: 30.10.2023 JP 2023185851
(71) Applicant: Toppan Holdings Inc., Tokyo 110-0016 (JP)
(72) Inventor: SUZUKI Yuta, Tokyo 110-0016 (JP)
(74) Representative: Plasseraud IP
(86) International application number: PCT/JP2024/037952
(87) International publication number: WO 2025/094815

(57) **Abstract**

A fluidic device includes a substrate having an upper surface in which a plurality of wells are formed; and a lid covering top of the wells, wherein the lid includes a cover part covering top of the wells, and a connecting part connecting between the cover part and the substrate; the connecting part has a lower surface welded to the upper surface of the substrate; and the lower surface of the connecting part is spaced apart from upper edge portions of the wells in a direction parallel to the upper surface of the substrate.

## Description

### [Technical Field]

The present invention relates to fluidic devices.

The present application claims the benefit of priority from Japanese Patent Application No. 2023-185851 filed in Japan on October 30, 2023, the contents of which are incorporated herein by reference.

### [Background Art]

Technologies for detecting biomolecules in fluidic devices are known. For example, in DNA microarray technologies, biomolecules are sometimes detected by introducing them into micropores and carrying out a reaction involving heating.

Also, technologies for single-molecule detection of biomolecules are known. Such technologies may include digital measurement technologies, such as the digital enzyme-linked immunosorbent assay (ELISA) described in Non-Patent Literature 1 (NPTL 1), digital polymerase chain reaction (PCR), and digital invasive cleavage assay (ICA).

For example Patent Literature 1 (PTL 1) discloses a fluidic device including a substrate with multiple microwells (depressions) formed in the upper surface thereof, and a lid having a lower end portion welded to the upper surface of the substrate.

In such a fluidic device, the lid is provided with a cover part covering the top of the multiple wells, and a connecting part connecting between the cover part and the substrate, with an internal space formed between the cover part and the substrate to function as a flow channel.

In order to detect biomolecules, an aqueous medium containing a target substance is supplied, first, to the flow channel of the fluidic device, and the aqueous medium is filled in the multiple wells provided in the flow channel.

Next, an oil-based sealing liquid is supplied to the flow channel to seal the aqueous medium in the multiple wells with the oil-based sealing liquid. Thus, the wells can function as multiple independent reaction spaces.

After that, the fluidic device is heated to heat the reaction solution and cause a detection reaction, thereby detecting the target substance.

### [Citation List]

### [Patent Literature]

PTL 1: WO2019/098301

### [Summary of the Invention]

### [Solution to Problem]

However, heating the fluidic device may form bubbles inside the fluidic device, and the bubbles formed inside may interfere with detection reactions and observations, and therefore improvements have been needed.

The present invention has been made in light of such circumstances and aims to provide a fluidic device capable of suppressing formation of bubbles and detecting biomolecules with high accuracy.

### [Solution to Problems]

The present inventor has observed a fluidic device that forms bubbles and has found that the bubbles are formed in the vicinities of the contact portion between the upper edge portions of the wells provided in the upper surface of the substrate and the lower surface of the connecting part of the lid.

The inventor has found thereafter that formation of bubbles can be suppressed by spacing the upper edge portions of the wells apart from the lower surface of the connecting part of the lid in the direction parallel to the upper surface of the substrate (in the horizontal direction) and thereby obtained the present invention.

An aspect of the present invention for solving the above issues encompasses the following modes.
[1] A fluidic device including a substrate having an upper surface in which a plurality of wells are formed; and a lid covering top of the wells, wherein the lid includes a cover part covering top of the wells, and a connecting part connecting between the cover part and the substrate; the connecting part has a lower surface welded to the upper surface of the substrate; and the lower surface of the connecting part is spaced apart from upper edge portions of the wells in a direction parallel to the upper surface of the substrate.
[2] The fluidic device according to [1], wherein a margin between the upper edge portions of the wells and the lower surface of the connecting part is 30 µm or more.
[3] The fluidic device according to [1] or [2], wherein a margin between the upper edge portions of the wells and the lower surface of the connecting part is 155 µm or more.
[4] The fluidic device according to any one of [1] to [3], wherein each of the wells has a volume of 1 fL or more and 6 nL or less.
[5] The fluidic device according to any one of [1] to [4], wherein a margin between the upper edge portions of the wells and the lower surface of the connecting part is 500 µm or less.
[6] The fluidic device according to any one of [1] to [5], wherein the connecting part is provided with a through-hole penetrating in a vertical direction in which the substrate and the lid are laminated; the through-hole defines an internal space between the substrate and the lid; and a well region, in which the wells are formed, is provided inside the internal space as viewed in the vertical direction.
[7] The fluidic device according to [6], wherein the wells are provided at regular intervals in the well region.
[8] The fluidic device according to [6], wherein the wells are provided at irregular intervals in the well region.
[9] The fluidic device according to any one of [6] to [8], wherein the lid is provided with an inlet and an outlet communicating with the internal space; and when a direction in which the inlet and the outlet are aligned is taken to be a longitudinal direction and a direction perpendicular to the longitudinal direction and the vertical direction is taken to be a lateral direction, a direction in which the wells are arrayed in the well region is different from the longitudinal direction and the lateral direction.
[10] The fluidic device according to any one of [6] to [9], wherein the connecting part includes an auxiliary connecting member provided between a plurality of the well regions and connecting between the cover part and the substrate.
[11] The fluidic device according to any one of [6] to [10], wherein the well region is surrounded by a welded portion formed by welding of the lower surface of the connecting part with the upper surface of the substrate.

### [Advantageous Effects of the Invention]

According to the present invention, there can be provided a fluidic device capable of suppressing formation of bubbles and detecting biomolecules with high accuracy.

### [Brief Description of the Drawings]

Fig. 1 is a schematic perspective view illustrating a fluidic device according to a preferred embodiment of the present invention.
Fig. 2 is a schematic longitudinal cross-sectional view illustrating the fluidic device taken along the line X-X of Fig. 1.
Fig. 3 is a schematic perspective view illustrating a lid from below.
Fig. 4 is a schematic longitudinal cross-sectional view illustrating a state in which a reagent solution is supplied into the internal space of a fluidic device.
Fig. 5 is a schematic longitudinal cross-sectional view illustrating a state in which a sealing liquid is supplied into the internal space of a fluidic device.
Fig. 6 is a schematic longitudinal cross-sectional view illustrating a state of the internal space that has been supplied with a sealing liquid.
Fig. 7 is a schematic longitudinal cross-sectional view illustrating a conventional fluidic device prone to bubble formation.
Fig. 8 is a schematic longitudinal cross-sectional view illustrating a state in which the substrate and the connecting member according to the embodiment shown in Fig. 1 are welded together.
Fig. 9 is a schematic plan view illustrating a state in which the lid has been detached from the fluidic device.
Fig. 10 is a schematic longitudinal cross-sectional view illustrating the fluidic device taken along the line Y-Y of Fig. 1.
Fig. 11 is a schematic plan view illustrating a state in which multiple wells are irregularly arranged.
Fig. 12 is a schematic plan view illustrating a state in which the direction in which multiple wells are arrayed is different from the direction in which the connecting member extends.
Fig. 13 is a schematic plan view illustrating a state in which a connecting member is welded at a position between wells in a substrate surface.
Fig. 14 is a schematic side view illustrating the shape of a well in a substrate according to an example.
Fig. 15 is a plan view image showing the vicinities of the wells of a substrate when a reagent solution is heated in a state in which the upper edge portions of the wells are in contact with the lower surface of the connecting member.
Fig. 16 is a plan view image showing the vicinities of the wells of a substrate when a reagent solution is heated in a state in which the upper edge portions of the wells are spaced 30 µm apart from the lower surface of the connecting member in the horizontal direction.
Fig. 17 is a plan view image showing the vicinities of the wells of a substrate when a reagent solution is heated in a state in which the upper edge portions of the wells are spaced 40 µm apart from the lower surface of the connecting member in the horizontal direction.
Fig. 18 is a plan view image showing the vicinities of the wells of a substrate when a reagent solution is heated in a state in which the upper edge portions of the wells are spaced 100 µm apart from the lower surface of the connecting member in the horizontal direction.
Fig. 19 is a plan view image showing the vicinities of the wells of a substrate when a reagent solution is heated in a state in which the upper edge portions of the wells are spaced 200 µm apart from the lower surface of the connecting member in the horizontal direction.

### [Description of the Embodiments]

With reference to the drawings where necessary, preferred embodiments of the present invention will be described in detail.

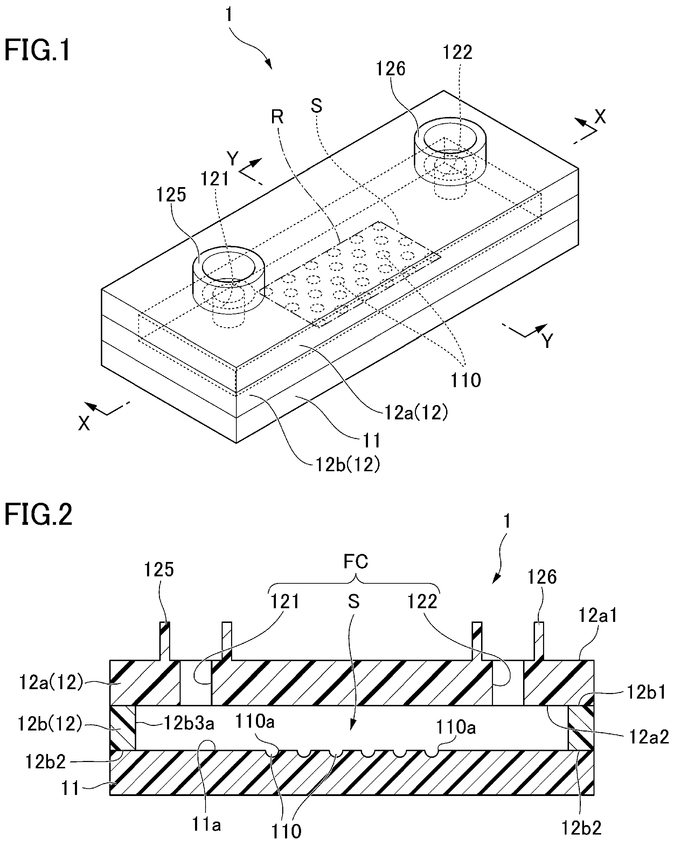

Fig. 1 is a schematic perspective view illustrating a fluidic device 1 according to a preferred embodiment of the present invention, and Fig. 2 is a schematic longitudinal cross-sectional view illustrating the fluidic device 1 taken along the line X-X of Fig. 1.

As shown in Figs. 1 and 2, the fluidic device 1 is a hollow device including a substrate 11 and a lid 12 covering one surface 11a of the substrate 11, with an internal space S defined between the substrate 11 and the lid 12. The fluidic device 1 is provided with an inlet 121 for charging a reagent solution into the internal space S and an outlet 122 for discharging the reagent solution. In the following description, the lid 12 side with respect to the substrate 11 is referred to as upper, the opposite side thereto is referred to as lower, and the direction in which the substrate 11 and the lid 12 are laminated is referred to as vertical direction. The direction toward the outlet 122 with respect to the inlet 121 is referred to as front, the opposite direction thereto is referred to as rear, and the direction in which the inlet 121 and the outlet 122 are aligned is referred to as longitudinal direction. The left side when facing forward is referred to as left, the opposite side thereto is referred to as right, and the direction perpendicular to the vertical and longitudinal directions is referred to as lateral direction.

The fluidic device 1 is configured such that it can detect a target substance that may be contained in the reagent solution supplied to the internal space S.

The reagent solution may contain detection reagents such as enzymes, substrates, nucleic acids, antibodies, antibody fragments, and fluorescent substances, other than biological or environmental samples that may contain target substances.

The types of the biological samples are not particularly limited, examples of which may include serum, plasma, urine, cell culture fluid, and PCR reaction solution.

The environmental samples may include, for example, river water, factory wastewater, etc.

The target substances include viruses, cells, and exosomes, other than DNA, RNA, and proteins as biomolecules, but are not limited thereto.

The substrate 11 is a substantially plate-like member made of a light-transmissive resin and having an upper surface in which multiple wells (depressions) 110 are formed. The wells 110 contain a reagent solution supplied to the internal space S, and function as reaction fields between a target substance that may be contained in the reagent solution and a detection reagent. Hereinafter, the rectangular region in which the wells 110 are formed is referred to as a well region R. The well region R is not limited to rectangular but may be circular, elliptical, or polygonal.

Although the details will be described later, the multiple wells 110 may be provided in the well region R at regular intervals, or may be arranged randomly. In the example shown in Fig. 1, the rows of three wells 110 aligned in the lateral direction are alternated with the rows of two wells 110 aligned in the lateral direction, so that the wells 110 are arranged at regular intervals. The well region R is provided at the center portion of the upper surface 11a with respect to the lateral and longitudinal directions. As viewed in the vertical direction, the well region R is provided so as not to overlap with the inlet 121 and the outlet 122. In other words, the well region R is provided between the inlet 121 and the outlet 122. Without being limited to the example in which the well region R is provided between the inlet 121 and the outlet 122, the wells 110 may only need to be provided inside the internal space S as viewed in the vertical direction. For example, as viewed in the vertical direction, the well region R may be provided at a position overlapped with the inlet 121 and the outlet 122.

The substrate 11 is preferably made of a resin that emits little autofluorescence. Examples of the resin that emits little autofluorescence include cycloolefin polymers, cycloolefin copolymers, silicones, polypropylenes, polycarbonates, polystyrenes, polyethylenes, polyvinyl acetates, fluororesins, and amorphous fluororesins.

The method of producing the substrate 11 is not particularly limited, but it can be produced, for example, using known injection molding, microimprinting technology, or nanoimprinting technology. The substrate 11 can also be produced by forming the wells 110 by etching using a known photolithography technology.

The thickness of the substrate 11 can be determined appropriately. The thickness of the substrate 11 may be larger than the depth of the wells 110, e.g., in the range of 0.6 mm to 5 mm. The thickness of the substrate 11 may preferably be in the range of 0.6 mm to 2 mm. When observing fluorescence from below the substrate 11 using a fluorescence microscope, the thickness of the substrate 11 may be, for example, 5 mm or less, preferably 2 mm or less, and more preferably 1.6 mm or less. This is because if the substrate 11 is excessively thick, the autofluorescence of the substrate 11 may increase, reducing the detection sensitivity.

The shape of the wells 110 is not particularly limited, but may be, for example, a hemispherical, columnar, prismatic, or frustum shape.

The size of the wells 110 is not particularly limited, but the wells 110 are preferably designed to have a size suitable for introducing one target substance to be detected into one well 110.

The volume of one well 110 is preferably 1 fL or more and 6 nL or less, more preferably 1 fL or more and 5 pL or less, even more preferably 1 fL or more and 2 pL or less, and most preferably 1 fL or more and 300 fL or less.

Designing the capacity of each well 110 in the above range, enzyme reactions, such as digital PCR and invader reactions, to be carried out in a microspace can be suitably carried out. Digital PCR can be used for detecting, for example, gene mutations.

The bottom of each well 110 may be flat, or may be curved (convex or concave).

The density of the wells 110 may be, for example, 100,000 to 10,000,000/cm², preferably 100,000 to 5,000,000/cm², and more preferably 100,000 to 1,000,000/cm². The wells 110 designed to have a density in the above range can facilitate the operation for encapsulating a reagent solution in a predetermined number of wells 110. It may also become easier to observe the wells for analysis of the experimental results.

The lid 12 includes a cover member (example of the cover part) 12a covering the top of the multiple wells 110, and a connecting member (example of the connecting part) 12b connecting between the cover member 12a and the substrate 11. The cover member 12a and the connecting member 12b are fixed to each other via an adhesive or by being welded together. The cover member 12a and the connecting member 12b may be formed integrally.

The material of the lid 12 is not particularly limited, but it is preferable to select a resin having low autofluorescence, e.g., a thermoplastic resin such as a cycloolefin polymer or cycloolefin copolymer.

The lid 12 is preferably made of a material that does not transmit light having wavelengths close to the wavelength detected during fluorescence observation of the target substance, and may be made of a thermoplastic resin to which carbon or metal particles, for example, are added in order to block such light. The method of producing the lid 12 is not particularly limited, but it can be produced, for example, using known injection molding.

The cover member 12a is provided with two through-holes penetrating in the thickness direction. The two through-holes are provided on a first side and a second side of the lid 12 in the length direction. Of the two through-holes, one is the inlet 121 used when charging liquids into the internal space S of the fluidic device 1, and the other is the outlet 122 used when discharging liquids from the internal space S. The liquids correspond to reagent solutions and sealing liquids.

The inlet 121, the internal space S, and the outlet 122 are in communication with each other and as a whole constitute a flow channel FC. As will be described in detail later, various liquids are supplied to the flow channel FC in a predetermined sequence, so that a target substance can be detected.

The cover member 12a has an upper surface 12a1 in which a cylindrical inlet port 125 is formed surrounding the inlet 121. The inlet port 125 is in communication with the inlet 121. The inlet port 125 is used for connecting a syringe to the inlet 121 when, for example, a liquid is filled in the internal space S using the syringe filled with the liquid.

The upper surface 12a1 of the lid 12 is provided with a cylindrical outlet port 126 surrounding the outlet 122. The outlet 126 is in communication with the outlet 122. The outlet port 126 is used for connecting a tube to the outlet 122 when, for example, a liquid is discharged from the internal space S.

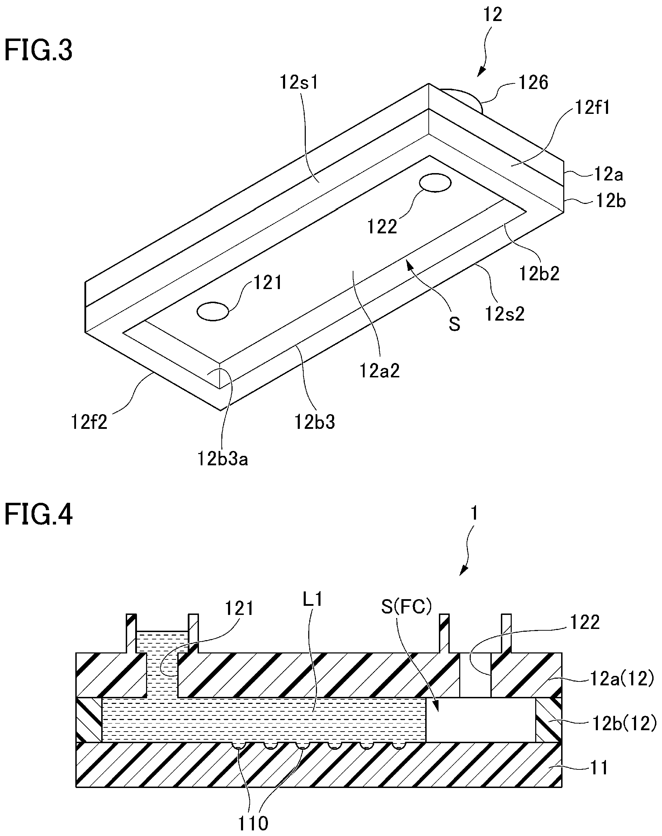

Fig. 3 is a schematic perspective view illustrating the lid 12 from below.

As shown in Fig. 3, the connecting member 12b forms a closed ring as viewed from the bottom, and is provided with a rectangular through-hole 12b3 at the center portion thereof in the longitudinal and lateral directions, penetrating in the vertical direction. The connecting member 12b includes a first wall portion 12s1 and a second wall portion 12s2 extending in the longitudinal direction and facing each other sandwiching the internal space S, and a third wall portion 12f1 and a fourth wall portion 12f2 extending in the lateral direction and facing each other sandwiching the internal space S. The first wall portion 12s1 is provided on the left side of the internal space S, and the second wall portion 12s2 is provided on the right side of the internal space S. The third wall portion 12f1 is provided in the front of the internal space S, and the fourth wall portion 12f2 is provided in the rear of the internal space S.

The connecting member 12b has an upper surface 12b1 fixed to a lower surface 12a2 of the cover member 12a.

The outer edge of the connecting member 12b is aligned with the outer edges of the cover member 12a and the substrate 11, and a lower surface (lower end portion) 12b2 of the connecting member 12b is welded and fixed to the upper surface 11a of the substrate 11 by laser light irradiation. Thus, as viewed in the vertical direction, the well region R is surrounded by the welded portion that has been formed by welding of the lower surface 12b2 of the connecting member 12b with the upper surface 11a of the substrate 11. The method of fixing the connecting member 12b to the substrate 11 is not limited to laser light irradiation, but may be a method using other welding that uses high frequency or ultrasound waves, etc.

As shown in Fig. 3, the inlet 121 and the outlet 122 of the cover member 12a are exposed due to the presence of the through-hole 12b3, and a liquid such as a reagent solution supplied through the inlet 121 is supplied to the inside of the through-hole 12b3, i.e., the internal space S.

The internal space S is defined by the lower surface 12a2 of the cover member 12a, the upper surface 11a of the substrate 11, and an inner surface 12b3a of the through-hole 12b3. The upper surface 11a of the substrate 11 and the lower surface 12a2 of the cover member 12a are horizontally parallel to each other.

The length of the connecting member 12b in the vertical direction, i.e., the height of the internal space S, is not particularly limited. For example, the height of the internal space S may be a height enabling passage of a reagent solution through the flow channel FC. The height of the internal space S may be, for example, 100 µm or less, preferably 50 µm or less, and more preferably around 30 µm. The height of the internal space S may be in the range of 20 µm to 30 µm.

The present embodiment has been described taking an example in which the lid 12 including the cover member 12a and the connecting member 12b is welded to the substrate 11 to constitute the fluidic device 1; however, this example is not limiting. For example, after welding the connecting member 12b and the substrate 11 together for fixation, the cover member 12a may be fixed to the connecting member 12b. Alternatively, the cover member 12a may be fixed to a member which has been obtained by integrally forming the substrate 11 and the connecting member 12b. Thus, even when the substrate 11 and the connecting member 12b are formed integrally, the portion of the connecting member 12b provided on the plane of the upper surface 11a of the substrate 11 in this integrated member in question is taken to be the lower surface 12b2 of the connecting member 12b.

Alternatively, the fluidic device 1 may be formed by laminating three separate bodies of the cover member 12a, the connecting member 12b, and the substrate 11, and simultaneously fixing between the cover member 12a and the connecting member 12b and between the connecting member 12b and the substrate 11.

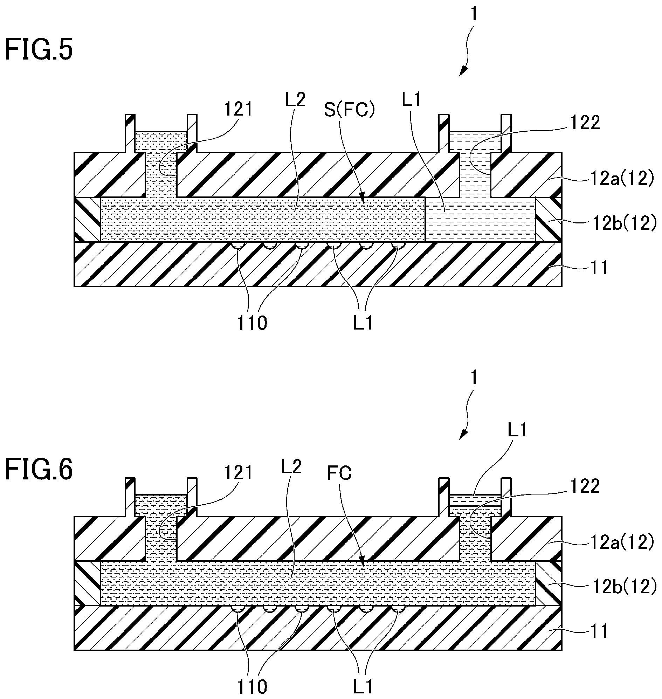

Fig. 4 is a schematic longitudinal cross-sectional view illustrating a state in which a reagent solution is supplied into the internal space S of the fluidic device 1. Fig. 5 is a schematic longitudinal cross-sectional view illustrating a state in which a sealing liquid is supplied into the internal space S of the fluidic device 1. Fig. 6 is a schematic longitudinal cross-sectional view illustrating a state of the internal space S that has been supplied with a sealing liquid L2.

In the fluidic device 1 constituted as described above, a target substance that may be contained in a reagent solution L1 can be detected by using the device as follows.

First, the reagent solution L1, which contains a sample diluted to a concentration of placing one target substance (one molecule in the case of molecules) in one well 110, is supplied to the internal space S via the inlet port 125 and the inlet 121.

Consequently, as shown in Fig. 4, the reagent solution L1 is transferred forward in the internal space S to fill the wells 110 with the reagent solution L1.

Next, the sealing liquid L2 is supplied to the internal space S via the inlet port 125 and the inlet 121. The sealing liquid L2 is preferably an oil.

Consequently, as shown in Fig. 5, the wells 110 are sealed with the sealing liquid L2 with the reagent solution L1 contained inside.

Examples of the oil used for the sealing liquid L2 include fluorine-based oils, silicone-based oils, hydrocarbon-based oils, and mixtures thereof. Specific examples of the sealing liquid include fluorine-based oils such as FC-40, FC-43, FC-770, FC-72 and FC-3283 (all manufactured by 3M), and silicone-based oils such as KF96 (manufactured by Shin-Etsu Chemical Co., Ltd.), NOVEC (model HFE-7500, manufactured by 3M), FLUO-OIL7500 (manufactured by Emulseo).

As shown in Fig. 6, after supplying the sealing liquid L2 until the internal space S is filled with the sealing liquid L2, the reagent solution L1 is left to stand until various reactions corresponding to the target substance therein are completed in the wells 110. In this case, the reagent solution L1 in the wells 110 is preferably heated. Thus, various reactions can be promoted. As a method of heating the reagent solution L1, for example, such a method may be used in which a heater is brought into contact with the substrate 11 from below to heat the reagent solution L1 via the substrate 11, but the method is not limited to this.

The target substance can be detected in the end by measuring fluorescence, etc. in the wells 110 from below via the substrate 11.

In the fluidic devices based on the conventional art, bubbles may form in the internal space in the process of heating the reagent solution mentioned above, and as a result may interfere with detection reactions and post-reaction observation.

Formation of bubbles in the fluidic devices based on the conventional art will be additionally described in detail below.

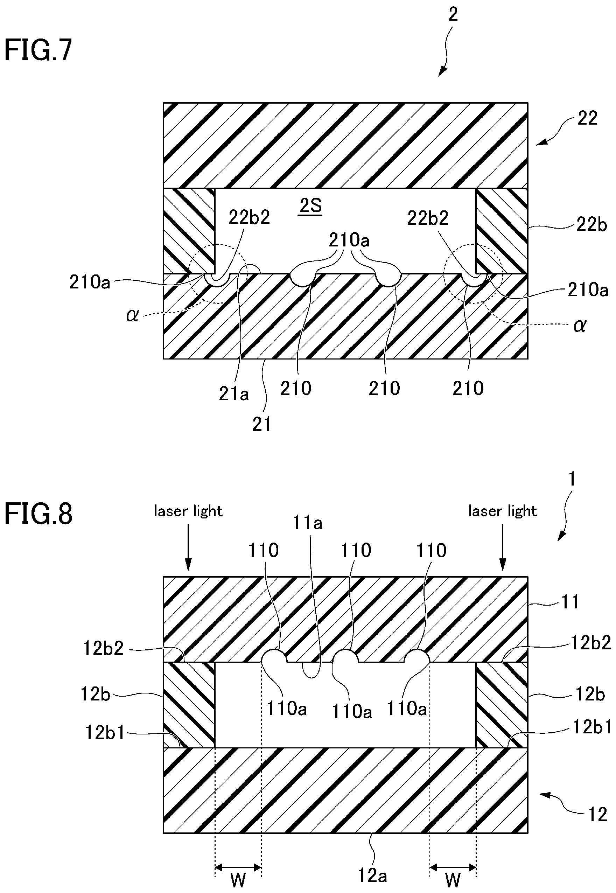

Fig. 7 is a schematic longitudinal cross-sectional view illustrating a conventional fluidic device 2 prone to bubble formation.

The present inventor has observed the fluidic device 2 prone to bubble formation, and found that, as shown in Fig. 7 at the position α, upper edge portions 210a of wells 210 formed in the upper surface of a substrate 21 are welded to a lower surface 22b2 of a connecting member 22b in a state of being in contact with each other (in other words, in a state in which the upper edge portions 210a of the wells 210 are overlapped with the connecting member 22b) and bubbles are formed in the vicinities of the contact portions.

Although not bound to any particular theory, if the upper edge portions 210a of the wells 210 are in contact with the connecting member 22b, it is considered that the wells 210 may be deformed in shape when welded. Consequently, it is estimated that when a reagent solution is supplied to an internal space 2S of the fluidic device 2, air may remain inside the wells 210, forming bubbles when the reagent solution is heated.

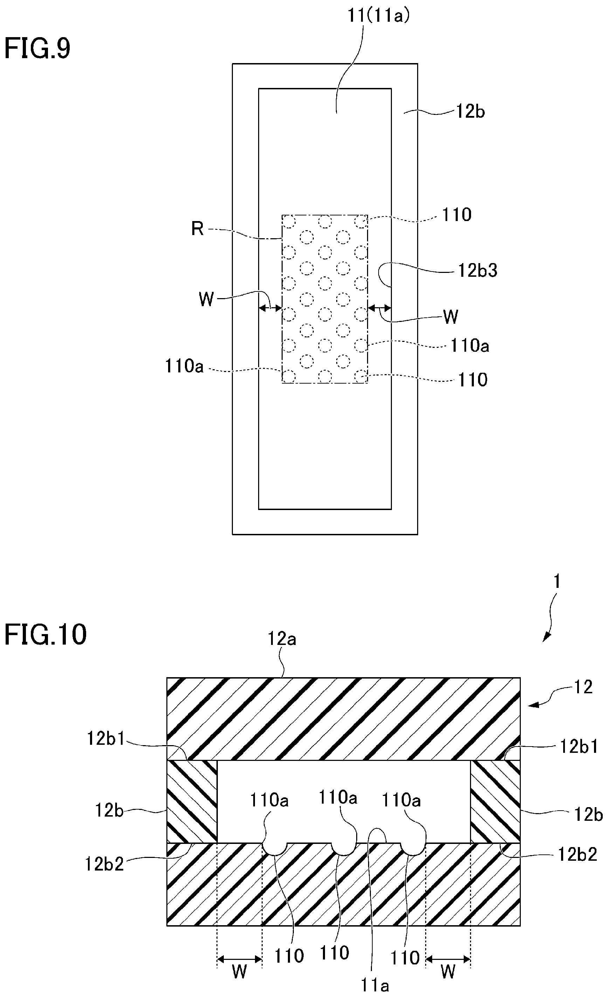

Fig. 8 is a schematic longitudinal cross-sectional view illustrating a state in which the substrate 11 and the connecting member 12b according to the embodiment shown in Fig. 1 are welded together. Fig. 9 is a schematic plan view illustrating a state in which the cover member 12a has been detached from the fluidic device 1, and Fig. 10 is a schematic longitudinal cross-sectional view illustrating the fluidic device 1 taken along the line Y-Y of Fig. 1.

In light of the bubble formation in the vicinities of the contact portions described above, in the present embodiment, no wells 110 are formed in portions of the upper surface 11a of the substrate 11, which are in contact with the connecting member 12b of the lid 12. In the process of producing the fluidic device 1, as shown in Fig. 8, the upside-down substrate 11 is placed on the lower surface 12b2 of the upside-down lid 12. After that, the substrate 11 and the lid 12 are welded together using laser light in the state in which the upper edge portions 110a of the wells 110 are not in contact with the lower surface 12b2 of the connecting member 12b.

Consequently, as shown in Fig. 9, none of the wells 110 located near the front, back, left and right edges of the substrate 11 are overlapped with the connecting member 12b of the lid 12 in plan view. Also, as shown in Fig. 10, for all of the wells 110, the entire upper edge portion 110a, which is circular in plan view, is spaced apart from the lower end portion (lower surface 12b2) of the connecting member 12b in the horizontal direction. The horizontal direction refers to the direction perpendicular to the vertical direction, in other words, refers to the direction parallel to the upper surface 11a of the substrate 11.

By spacing the lower surface 12b2 of the connecting member 12b apart from the upper edge portions 110a of the wells 110 in the horizontal direction, air is prevented from remaining inside the wells 110 during supply of the reagent solution L1 to effectively suppress formation of bubbles during heating.

As will be shown later in examples, a margin W in the horizontal direction between the upper edge portions 110a of the wells 110 and the lower surface 12b2 of the connecting member 12b is preferably 30 µm or more, and more preferably 155 µm or more.

If the upper edge portions 110a of the wells 110 are spaced apart from the lower surface 12b2 of the connecting member 12b by 30 µm or more, excessive heat transmission to the wells 110 can be prevented during welding, and thus formation of bubbles in the process of heating the reagent solution can be effectively suppressed.

If the upper edge portions 110a of the wells 110 are spaced apart from the lower surface 12b2 of the connecting member 12b by 155 µm or more, the state in which the upper edge portions 110a of the wells 110 are spaced apart from the lower surface 12b2 of the connecting member 12b can be ensured in all the products including part of the products in which either or both of the substrate 11 and the connecting member 12b have been displaced by some chance in the horizontal direction due to vibration, etc. during welding, or in which, from the perspective of processing accuracy, the substrate 11 or the lid 12 with dimensions slightly different from the designed values has been produced.

The margin W is preferably 500 µm or less. If the margin W is larger than 500 µm, the well region R having an area for arranging the wells 110 is reduced to reduce the number of wells to be arranged, which may deteriorate detection accuracy.

The margin W may satisfy the above conditions throughout the circumference of the well region R; however, it is particularly preferable that the margin W is 30 µm or more and 500 µm or less in the lateral direction. The range of 30 µm or more and 500 µm or less may be satisfied only by the margin W in the lateral direction which is between the upper edge portions 110a of the wells 110 and the lower surface 12b2 of the first and second wall portions 12s1 and 12s2 of the connecting member 12b, and the distance in the longitudinal direction which is between the upper edge portions 110a of the wells 110 and the lower surface 12b2 of the third and fourth wall portions 12f1 and 12f2 of the connecting member 12b may exceed the above range of the margin W. For example, this is because there are cases where the wells 110 are not formed at positions overlapping with the inlet 121 and the outlet 122 as viewed in the vertical direction.

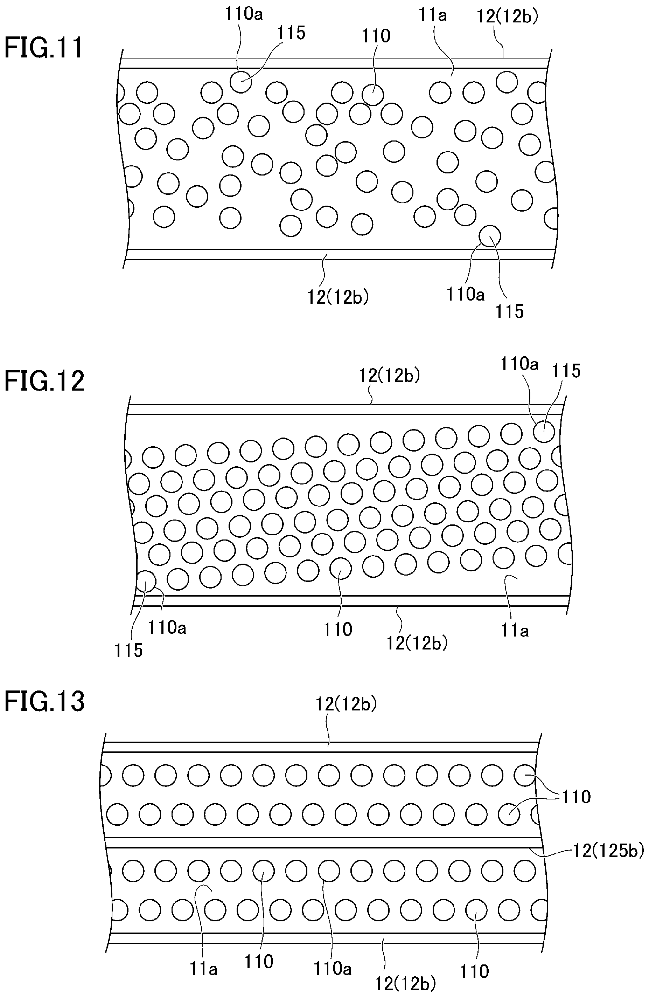

Figs. 11 to 13 are schematic plan views illustrating arrangement variations of wells 110 and 115 on the substrate 11.

Specifically, Fig. 11 is a schematic plan view illustrating a state in which multiple wells 110 and 115 are arranged irregularly, Fig. 12 is a schematic plan view illustrating a state in which the direction in which the multiple wells 110 and 115 are arrayed is different from the direction in which the connecting member 12b extends, and Fig. 13 is a schematic plan view illustrating a state in which the connecting member 12b is welded at a position between the wells 110 in the substrate surface (11a).

As shown in Fig. 11, even when the multiple wells 110 and 115 are arranged irregularly, the upper edge portions 110a of the wells 115 closest to the lower surface of the connecting member 12b are spaced apart from the lower surface of the connecting member 12b in the horizontal direction to suppress formation of bubbles.

As shown in Fig. 12, even when the direction in which the multiple wells 110 and 115 are arranged is different from the direction in which the connecting member 12b extends (longitudinal direction and/or lateral direction), the upper edge portions 110a of the wells 115 closest to the lower surface of the connecting member 12b are spaced apart from the lower surface of the connecting member 12b in the horizontal direction to suppress formation of bubbles.

In the embodiment shown in Fig. 13, the multiple wells 110 are arrayed in rows in the length direction (longitudinal direction) in the substrate upper surface 11a, and multiple rows of the wells 110 are formed in the lateral direction. Between the rows of the wells 110, gaps are formed in the short-side direction (lateral direction). In the example shown in Fig. 13, two well regions R are provided in each of which there is a group of one or more rows of the wells 110, and a gap is provided between the two well regions R.

The lid 12 further includes an auxiliary connecting member 125b at the center portion thereof in the lateral direction to connect between the cover member 12a and the substrate 11.

The lower surface of the auxiliary connecting member 125b located at the center portion of the connecting member 12b in the short-side direction is welded in the gap located at the center portion of the substrate surface 11a in the lateral direction (short-side direction). The upper surface of the auxiliary connecting member 125b is fixed to the lower surface of the cover member 12a at the center portion thereof in the lateral direction. The lower surface 12b2 of the auxiliary connecting member 125b is spaced apart from the upper edge portions 110a of the wells 110 in the direction parallel to the upper surface 11a of the substrate 11.

The auxiliary connecting member 125b preferably extends parallel to the flow channel FC. Thus, in the example shown in Fig. 13, the auxiliary connecting member 125b extends in the longitudinal direction; however, without being limited to this example, the auxiliary connecting member 125b may extend in the direction perpendicular to the longitudinal direction.

In this way, even in the case where the auxiliary connecting member 125b is arranged at a position other than the edge portions of the fluidic device in the horizontal direction, the lower surface of the auxiliary connecting member 125b can be spaced apart from the upper edge portions 110a of the wells 110 to thereby suppress formation of bubbles.

Preferred embodiments have so far been specifically described; however, the present invention should not be construed as being limited to these embodiments but may be variously modified within the scope of the present invention recited in the claims.

For example, in the embodiments shown in Figs. 1 to 13, the cover member 12a of the lid 12 and the connecting member 12b are separately formed; however, they may be formed integrally. In this case, the portion of the lid covering the multiple wells is a cover part, and the portion connecting between the cover part and the substrate is a connecting part.

In the embodiments shown in Figs. 1 to 10, the liquid discharged from the outlet port 126 is designed to spread throughout the fluidic device 1; however, a wall may be provided along the edge portion of the upper surface 12a1 of the cover member 12a so that the liquid can be collected on the cover member 12a.

### Examples

The present invention will be described below using examples, but the present invention should not be limited to these examples.

In the present experiment, the upper edge portions of the wells 110 of the substrate 11 shown in Fig. 2 were spaced apart from the lower surface of the connecting member 12b of the lid 12 in the horizontal direction to examine whether formation of bubbles could be suppressed during heating of the reagent solution.

The present experiment used a fluidic device obtained by welding the substrate 11 and the connecting member 12b together using laser light.

The substrate 11 and the lid 12 used were made of a cycloolefin polymer ZEONOR (R) 1020R manufactured by Zeon Corporation. The lid 12 was colored black by mixing in carbon black.

As a reagent solution, an enzyme solution containing 20 mM (mol/L) NaCl, 25 mM MgCl₂, 50 mM tris-HCl buffer solution (pH 8.5), 0.05% surfactant, 0.2 mg/mL enzyme, and water was used. The reagent solution was heated via the substrate 11 at 66°C for 25 minutes.

As the surfactant, Tween (trademark) 20, for example, can be used.

As a sealant liquid, a silicone oil KF-96 manufactured by Shin-Etsu Chemical Co., Ltd. was used.

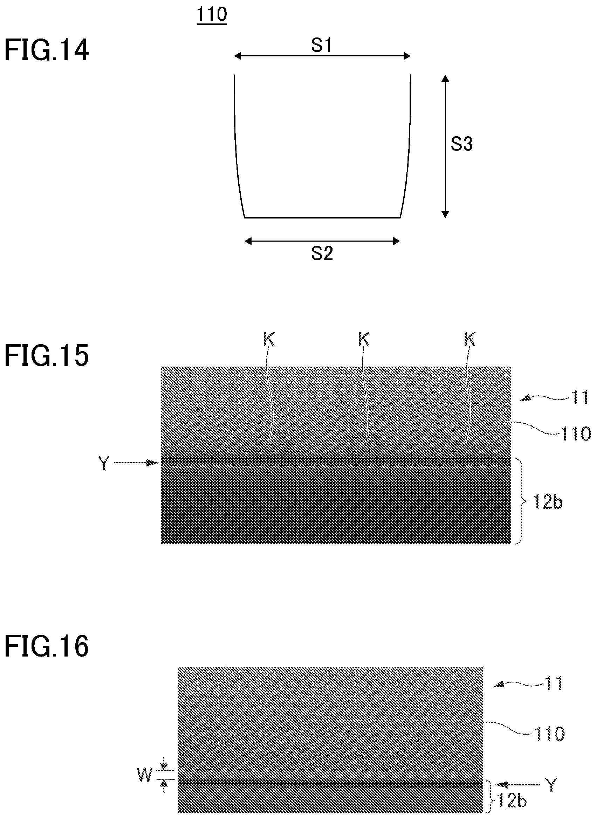

Fig. 14 is a schematic side view illustrating the shape of the wells 110 in the substrate 11 according to an example. The wells 110 had a columnar shape with the diameter gradually decreasing downward from the top, and had a diameter S1 of 12.5 µm at the upper edge portion, a diameter S2 of 11.0 µm at the bottom, and a depth S3 of 10 µm. The interval between the wells 110 (the distance between the upper edge portions of the wells 110) was 2.5 µm. The substrate 11 was provided with about 1,000,000 wells 110 each having the above shape and dimensions.

Fig. 15 is a plan view image showing the vicinities of the wells 110 of the substrate 11 when the reagent solution was heated in a state in which the upper edge portions of the wells 110 were in contact with the lower surface of the connecting member 12b. The overlap between the wells 110 and the connecting member 12b in the horizontal direction was about 5 µm to 6 µm.

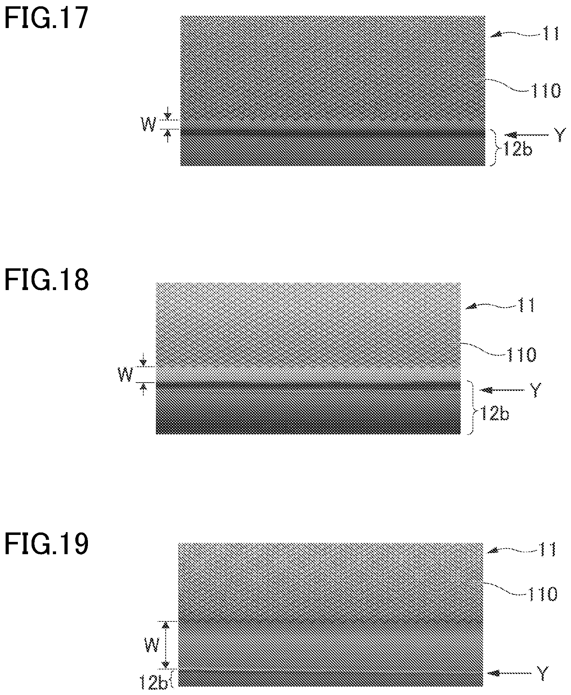

Fig. 16 is a plan view image showing the vicinities of the wells 110 of the substrate 11 when the reagent solution was heated in a state in which the upper edge portions of the wells 110 were spaced 30 µm apart from the lower surface of the connecting member 12b in the horizontal direction. Fig. 17 is a plan view image showing the vicinities of the wells 110 of the substrate 11 when the reagent solution was heated in a state in which the upper edge portions of the wells 110 were spaced 40 µm apart from the lower surface of the connecting member 12b in the horizontal direction. Fig. 18 is a plan view image showing the vicinities of the wells 110 of the substrate 11 when the reagent solution was heated in a state in which the upper edge portions of the wells 110 were spaced 100 µm apart from the lower surface of the connecting member 12b in the horizontal direction. Fig. 19 is a plan view image showing the vicinities of the wells 110 of the substrate 11 when the reagent solution was heated in a state in which the upper edge portions of the wells 110 were spaced 200 µm apart from the lower surface of the connecting member 12b in the horizontal direction.

In Figs. 16 to 19, the margin between the upper edge portions of the wells 110 and the lower surface of the connecting member 12b is indicated by W.

The images shown in Figs. 15 to 19 were captured using an All-in-One Fluorescence Microscope BZ-X810 manufactured by Keyence Corporation. The images of the inside of the fluidic device were captured from above via the cover member 12a of the lid 12 (see Fig. 2).

When the reagent solution was heated in a state in which the upper edge portions of the wells 110 of the substrate 11 were in contact with the lower surface of the connecting member 12b, multiple bubbles K were formed, as shown in Fig. 15, in the vicinity of a welded portion Y between the substrate 11 and the connecting member 12b.

In this regard, when the reagent solution was heated in a state in which the upper edge portions of the wells 110 of the substrate 11 were spaced apart from the lower surface of the connecting member 12b in the horizontal direction, no bubbles K were formed as shown in Figs. 16 to 19.

As described so far, it was confirmed that formation of the bubbles K could be effectively suppressed by welding the substrate 11 and the connecting member (connecting part) together in a state in which the upper edge portions of the wells 110 of the substrate 11 were spaced apart from the lower surface of the connecting member 12b in the horizontal direction (in the direction parallel to the upper surface of the substrate 11).

### [Reference Signs List]

1 ... Fluidic device
11 ... Substrate
12 ... Lid
12a ... Cover member (example of cover part)
12b ... Connecting member (example of connecting part)
12b2 ... Lower surface (lower end portion)
125b ... Auxiliary connecting member
110 ... Well (microwell)
110a ... Upper edge portion
121 ... Inlet
K ... Bubble
122 ... Outlet
L1 ... Reagent solution
L2 ... Sealing liquid
S ... Internal space
S1 ... Diameter of well at upper edge portion
S2 ... Diameter of well at bottom
S3 ... Depth of well
W ... Margin between upper edge portions of wells and lower surface of connecting member (example of connecting part)

## Claims

1. A fluidic device comprising
a substrate having an upper surface in which a plurality of wells are formed; and
a lid covering top of the wells, wherein
the lid includes
a cover part covering top of the wells, and
a connecting part connecting between the cover part and the substrate;
the connecting part has a lower surface welded to the upper surface of the substrate; and
the lower surface of the connecting part is spaced apart from upper edge portions of the wells in a direction parallel to the upper surface of the substrate.

2. The fluidic device according to claim 1, wherein a margin between the upper edge portions of the wells and the lower surface of the connecting part is 30 µm or more.

3. The fluidic device according to claim 2, wherein a margin between the upper edge portions of the wells and the lower surface of the connecting part is 155 µm or more.

4. The fluidic device according to any one of claims 1 to 3, wherein each of the wells has a volume of 1 fL or more and 6 nL or less.

5. The fluidic device according to any one of claims 1 to 3, wherein a margin between the upper edge portions of the wells and the lower surface of the connecting part is 500 µm or less.

6. The fluidic device according to claim 1, wherein
the connecting part is provided with a through-hole penetrating in a vertical direction in which the substrate and the lid are laminated;
the through-hole defines an internal space between the substrate and the lid; and
a well region, in which the wells are formed, is provided inside the internal space as viewed in the vertical direction.

7. The fluidic device according to claim 6, wherein the wells are provided at regular intervals in the well region.

8. The fluidic device according to claim 6, wherein the wells are provided at irregular intervals in the well region.

9. The fluidic device according to any one of claims 6 to 8, wherein
the lid is provided with an inlet and an outlet communicating with the internal space; and
when a direction in which the inlet and the outlet are aligned is taken to be a longitudinal direction and a direction perpendicular to the longitudinal direction and the vertical direction is taken to be a lateral direction, a direction in which the wells are arrayed in the well region is different from the longitudinal direction and the lateral direction.

10. The fluidic device according to any one of claims 6 to 8, wherein the connecting part includes an auxiliary connecting member provided between a plurality of the well regions and connecting between the cover part and the substrate.

11. The fluidic device according to any one of claims 6 to 8, wherein the well region is surrounded by a welded portion formed by welding of the lower surface of the connecting part with the upper surface of the substrate.
